# EUROPÄISCHE PATENTSCHRIFT

(11) **EP 1 255 725 B1**
(45) Veröffentlichungstag und Bekanntmachung des Hinweises auf die Patenterteilung: **13.01.2010**
(21) Anmeldenummer: 01911547.6
(22) Anmeldetag: 29.01.2001
(51) Int. Cl.: C07C 229/00

(54) **5-AMINOLÄVULINSÄURE-FORMULIERUNG IN NICHTWÄSSRIGEN LÖSUNGSMITTELN**
5-AMINOLEVULINIC ACID FORMULATION DISSOLVED/DISPERSED IN NON-AQUEOUS SOLVENTS
FORMULATION D'ACIDE 5-AMINOLEVULINIQUE DISSOUS OU DISPERSE DANS DES SOLVANTS NON AQUEUX

(30) Priorität: 28.01.2000 DE 10003620
(43) Veröffentlichungstag der Anmeldung: 13.11.2002
(73) Patentinhaber: Biofrontera Bioscience GmbH, 51377 Leverkusen (DE)
(72) Erfinder: GANDER, Bruno, CH-6405 Immensee (CH); BUNKE, Antje, 01259 Dresden (DE); BURMEISTER, Gerd, CH-6414 Oberarth (CH)
(74) Vertreter: Dey, Michael
(86) Internationale Anmeldenummer: PCT/EP2001/000930
(87) Internationale Veröffentlichungsnummer: WO 2001/055092

(56) Entgegenhaltungen:
- EP-A- 0 633 017
- EP-A- 0 704 209
- WO-A-95/05813
- WO-A-96/28412
- WO-A-99/22802
- WO-A-99/53962
- MALIK ET AL: "Topical application of 5-aminolevulinic acid, DMSO and EDTA: protoporphyrin IX accumulation in skin and tumors of mice" STN CHEMICAL ABSTRACTS,XX,XX, Bd. 12, Nr. 123, 18. September 1995 (1995-09-18), XP002077405

## Beschreibung

Die Erfindung betrifft wasserfreie Zusammensetzungen, die 5-Aminolävulinsäure oder/und Derivate davon gelöst oder dispergiert in einer nichtwässrigen Flüssigkeit enthalten sowie ein Doppelkammersystem, welches eine nichtwässrige 5-Aminolävulinsäure-Formulierung in einer ersten Kammer sowie ein wässriges Trägersystem in einer zweiten Kammer umfasst.

Die photodynamische Therapie ist eine neue und vielversprechende Methode zur Behandlung von verschiedenen prämalignen und malignen Erkrankungen, die mit der Zellproliferation in Zusammenhang stehen. Das Prinzip der photodynamischen Therapie beruht darauf, einen sog. Photosensibilisator in das Tumorgewebe einzubringen und diesen durch Bestrahlung mit Licht geeigneter Wellenlänge in einem cytotoxisch aktiven Wirkstoff umzuwandeln, welcher letzlich die Zerstörung der Zellen bewirkt. Die Selektivität dieser Methode beruht in einer stärkeren Anreicherung des Sensibilisators in schnell proliferierenden Tumorzellen im Vergleich zum Normalgewebe. Durch eine lokal begrenzte Lichtbestrahlung kann der in den Tumorzellen enthaltende Sensibilisator gezielt aktiviert werden, wodurch die Krebszellen unter weitgehender Schonung des gesunden Gewebes zerstört werden.

Bisher wurde als Photosensibilisator zumeist ein intravenös verabreichbares Gemisch von Haematoporphyrinderivanten verwendet. Trotz ermutigender klinischer Erfolge bei unterschiedlichen Krebsarten weisen diese Haematoporphyrinderivate jedoch verschiedene Nachteile auf. Erstens treten infolge der geringen Tumorselektivität und der nur langsamen Elimination aus dem Körper relativ hohe Wirkstoffkonzentrationen im Normalgewebe auf. Demzufolge finden bei der Bestrahlung unerwünschte photochemische Reaktionen im gesunden Gewebe statt. Zweitens resultiert diese Behandlung in einer allgemeinen Lichtempfindlichkeit, so dass sich der Patient während etwa vier Wochen nicht dem Tageslicht aussetzen darf.

Eine Verringerung der hohen Wirkstoffkonzentration im Normalgewebe und somit der unerwünschten Nebenwirkungen kann in bestimmten Fällen, insbesondere bei dermatologischen und gynäkologischen Anwendungen durch die Entwicklung topisch applizierbarer Wirkstofformulierungen anstelle der bekannten systemischen Formulierungen erreicht werden. WO95/05813 beschreibt beispielsweise ein mit 5-ALA imprägniertes Pflaster für die dermale Applikation. Zur Verringerung der Lichtempfindlichkeit wird weiterhin versucht, Precursoren von Photosensiblisatoren einzusetzen, die photochemisch inaktiv sind und erst innerhalb der Zielzelle in einen Photosensibilisator umgewandelt werden.

5-Aminolävulinsäure ist eine körpereigene Substanz, die aus Glycin und Succinyl-CoA synthetisiert wird. Im Rahmen der Haem-Biosynthese entsteht aus 5-Aminolävulinsäure (5-ALA) in mehreren schnell verlaufenden Reaktionsschritten das hochgradig photoaktive Protophorphyrin IX, das anschließend in einer langsamen Reaktion zur Haem umgewandelt wird. Ein natürlicher Regelmechanismus hemmt bei zu hoher Haemkonzentration sowohl die körpereigene Synthese von 5-ALA als auch den Abbau von Protoporphyrin IX.

Durch die exogene Verabreichung von synthetisch hergestellter 5-ALA wird dieser Regelmechanismus umgangen, was zu einer erhöhten Produktion von Protoporphyrin IX führt. Da dessen Abbau durch den natürlichen Kontrollmechanismus weiterhin gehemmt ist, reichert sich das Protoporphyrin IX in den Zellen an. Protoporphyrin IX kann bei Bestrahlung mit Licht eine photochemische Oxidationsreaktion eingehen und wirkt somit als Photosensibiliasator. Bei Absorption eins Lichtquants durch das Sensibilisatormolekül wird dieses zunächst in einen elektronisch angeregten Zustand (Singulettzustand) versetzt, welcher relativ kurzlebig ist und seine Überschußenergie entweder innerhalb einer Nanosekunden durch Emission eines Fluoreszenzphotons wieder abgibt oder in einen relativ langlebigen Triplettzustand übergeht. Aus diesem Triplettzustand kann Energie auf in der Zelle vorhandene Sauerstoffmoleküle übertragen werden. Der dabei entstehende Singulett-Sauerstoff wirkt cytotoxisch, insbesondere auf prolifierierende Zellen, da er mit Zellkomponenten, z.B. der Zellmembran und Mitochondrien, reagiert oder die Bildung von zellschädigenden Radikalen auslöst. Weiterhin führt die Bestrahlung des Photosensibilisators zu einer charakteristischen Fluoreszenzstrahlung, welche für Nachweisreaktionen, beispielsweise zum Nachweis proliferierender Zellen verwendet werden kann.

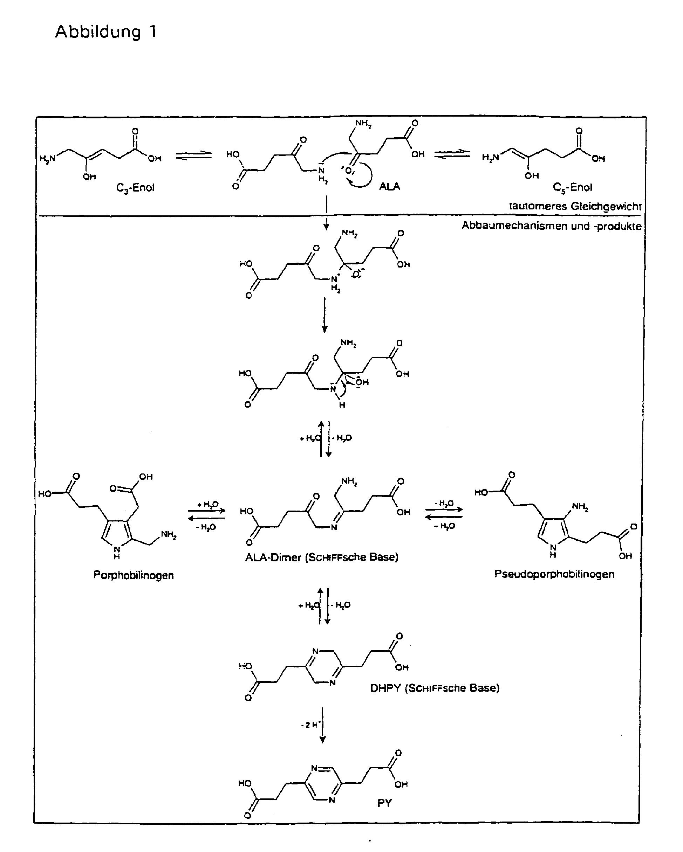

Bei 5-ALA handelt es sich um eine chemisch äußerst instabile Substanz, die einem breiten Spektrum von Zersetzungsreaktionen unterliegt (siehe z.B. Granick und Mauzerall, J. Biol. Chem. 232 (1958), 1119-1140; Franck und Stratmann, Heterocycles 15 (1991), 919-323, Jaffe und Rajagopalan, Bioorg. Chem. 18 (1990), 381-394; Butler und George, Tetrahedron 48 (1992), 7879-7886; Novo et al., J. Photochem. Photobiol. B: Biol. 34 (1996), 143-148; Scott, Biochem. J. 62 (1955), 6 P; Dalton et al. Pharm. Res. 16 (1999), 288-295). Diese Zersetzungsreaktionen sind schematisch in Abbildung 1 dargestellt. Als α-Aminoketon bildet 5-ALA eine dimere Schiffsche Base (DHPY), die leicht zur aromatischen Verbindung PY oxidiert wird. Als Nebenreaktionen können die Umsetzungen zu Porphobilinogen bzw. Pseudoporphobilinogen auftreten. Der erste Reaktionsschritt aller Abbaustufen, die Bildung der Schiff'schen Base über die in Abbildung 1 dargestellten instabilen Zwischenstufen, ist ein stark pHabhängiges Gleichgewicht, wobei höhere pH-Werte von z.B. oberhalb pH 5 die 5-ALA-Zersetzung beschleunigen. Als hinreichend stabil erweist sich lediglich eine saure wässrige Lösung von 5-ALA HCl. Die pH-Wert-Optimierung eignet sich jedoch nicht als Mittel zur Stabilisierung von 5-ALA als Arzneimittel, da ein stark saures Medium nicht therapeutisch eingesetzt werden kann.

Neben der Instabilität von 5-ALA stellt ihr ausgeprägter ionischer Charakter ein Problem bezüglich der Bioverfügbarkeit dar. 5-ALA liegt im physiologisch tolerierbaren pH-Vereich (pH 5 bis 8) als Zwitterion vor, d.h. mit dissoziierter Carboxylgruppe und mit protonierter Aminogruppe. Bekanntermaßen sind derart geladene Substanzen schlecht membrangängig, d.h. sie werden nur in geringem Ausmaß durch Epithelien und durch Zellmembranen transportiert. Damit ist die Bioverfügbarkeit gering. Dies erklärt auch die Tatsache, daß 5-ALA in den bisherigen klinischen Anwendungen in sehr hohen Dosen eingesetzt werden musste.

Aufgabe der vorliegenden Erfindung war es somit, 5-ALA enthaltende Zusammensetzungen bereitzustellen, bei denen die aus dem Stand der Technik bekannten Nachteile zumindest teilweise beseitigt sind und die insbesondere eine verbesserte chemische Stabilität und eine bessere Membrangängigkeit besitzen.

Gelöst wird diese Aufgabe durch Einbringen von 5-ALA, oder/und Derivaten davon in nichtwässrige Flüssigkeiten mit einer Dielektrizitätskonstante ε < 80 bei 25 °C, wobei diese Flüssigkeiten vorzugsweise physiologisch verträglich und mit Wasser mischbar sind. Beispiele solcher Flüssigkeiten sind 1,2-Propylenglykol und Glycerin.

Ein Gegenstand der Erfindung ist somit eine wasserfreie Zusammensetzung, die eine Wirksubstanz ausgewählt aus 5-ALA oder/und einem Derivat davon gelöst oder dispergiert in einer nichtwässrigen Flüssigkeit enthält, die eine Dielektrizitätskonstante ε von weniger als 80 bei 25 °C aufweist, welche eine Stabilisierung der 5-Aminolävulinsäure (5-Ala) oder/und dem Derivat davon bewirkt.

Erfindungsgemäß umfasst die Zusammensetzung eine Wirksubstanz ausgewählt aus 5-Aminolävulinsäure oder/und einem Derivat davon. Unter "Derivat" sind insbesondere Salze, Ester, Komplexe und Additionsverbindungen zu verstehen. Besonders bevorzugt ist die Wirksubstanz 5-Aminolävulinsäure oder ein Salz oder Ester davon. Bevorzugte Beispiele für Salze und Ester sind 5-ALA-Hydrochlorid, -Sulfat, -Nitrat, -Phosphat, -Borat, -Tannat, -Lactat, -Glycolat, -Succinat, -Citrat, -Tartrat, -Embonat, bzw. 5-ALA-Methylat, -Ethylat, Propionat, -Butyrat, -Hexanoat, -Octoanat, -Dodecanoat, -Myristat, -Palmitat, -Oleat.

Aufgrund der Lösung bzw. Dispergierung in nichtwässrigen Flüssigkeiten liegt die Wirksubstanz 5-ALA vorzugsweise zumindest teilweise in einer Enolform vor, die in gegenüber Wasser weniger polaren Flüssigkeiten verstärkt gebildet wird. Das Vorhandensein der Enolform führt zu einer Gelbfärbung der Zusammensetzung, die jedoch nicht auf eine Zersetzung 5-ALA zu einem der in Abbildung 1 gezeigten Abbauprodukte zurückzuführen ist. Die Bildung der Enolform bewirkt eine Stabilisierung von 5-ALA, durch welche die Entstehung der Schiff'schen Base gemäß Abbildung 1 und in Folge dessen auch die Reaktion zu weiteren Abbauprodukten verzögert werden kann. Zudem wird die verglichen mit der Ketoform weniger polare Enolform der 5-ALA besser durch physiologische Membranen aufgenommen. Somit kann neben der chemischen Stabilisierung auch eine verbesserte Bioverfügbarkeit erzielt werden.

Bevorzugte Beispiele für nichtwässrige Flüssigkeiten, die zur Lösung bw. Dispergierung des Wirkstoffes eingesetzt werden, sind pharmazeutisch verträgliche Lösungsmittel wie Alkohole, z.B. höhere Alkohole, wie etwa C₁-C₂₀ Alkohole, Ether und Ester, mehrwertige, z.B. zwei- oder dreiwertige Alkohole und deren Ester, z.B. Glycerin und dessen Mono-, Di- und Triester mit C₁-C₂₀-Carbonsäuren, 1,2-Propylenglykol, 1,3-Propylenglykol und deren Mono- und Diester mit C₁-C₂₀ Carbonsäuren, Poly(alkylenoxide), insbesondere Poly(ethylen- oder/und propylenoxide) mit bis zu 1000 Alkyleneinheiten und deren Ester, Phospholipide, Ester höherer Carbonsäuren, Sulfoxide wie etwa Dimethylsulfoxid (DMSO), N-Vinylpyrrolidon und N,N-Dimethylacetamid. Ebenso geeignet sind Mischungen von zwei oder mehreren der genannten Substanzen. In manchen Ausführungsformen kann es bevorzugt sein, wenn es sich bei der nichtwässrigen Flüssigkeit nicht um ein Alkandiol, ein Alkantriol oder um eine organische Säure handelt.

Weiterhin kann die Zusammensetzung Substanzen enthalten, die zur Verfestigung der Formulierung von 5-ALA, z.B. als Salz oder in Form von Estern bei geringer Temperatur, z.B. Kühlschranktemperaturen, dienen, welche sich bei Raum- bzw. Körpertemperatur wieder verflüssigen und somit zu einer weiteren Erhöhung der Lagerstabilität von 5-ALA beitragen können. Beispiele für solche Substanzen mit temperaturabhängigem Fest-Flüssig-Verhalten sind Pflanzenöle wie Baumwollsamenöl, Erdnussöl, Sesamöl, Tenside wie Cremophor^{®} EL, PEG 400-Monostearat, PEG 600-Monostearat, Polysorbat (Tween 61) und Lösungsvermittler wie Solutol^{®} HS15, Isopropylmyristat und -palmitat. Diese Substanzen sind als solche größtenteils keine guten Lösungsmittel für 5-ALA, können jedoch in Kombination mit anderen der genannten Substanzen, z.B. Glycerin oder Propylenglykol, die 5-ALA bzw. deren Derivate jedoch in Lösung halten, und darüber hinaus der Zusammensetzung die Eigenschaft vermitteln, sich bei geringer Temperatur zu verfestigen und sich bei Umgebungstemperatur wieder zu verflüssigen.

Die Zusammennsetzung kann darüber hinaus gegebenenfalls zusätzlich Wasser oder eine wässrige Lösung, vorzugweise in geringen Mengen von maximal bis zu 50 Gew.-%, besonders bevorzugt von maximal bis zu 25 Gew.-% bezogen auf das Gesamtgewicht der Zusammensetzung enthalten. Die Zugabe von Wasser erfolgt vorzugsweise erst unmittelbar vor der beabsichtigten Anwendung der Zusammensetzung.

Der Anteil der Wirksubstanz, z.B. 5-ALA, in der Zusammensetzung hängt im wesentlichen von dem vorgesehenen Anwendungszweck ab. Im allgemeinen sind etwa 1 bis 25 Gew.-%, bezogen auf das Gesamtgewicht der Zusammensetzung, vorhanden. Höhere bzw. niedrigere Dosierungen sind jedoch machbar. Für Anwendungen im Zusammenhang mit photodynamischer Therapie hat sich ein Anteil von 1 bis 15 Gew-%, insbesondere von etwa 2 bis 10 Gew.-% als geeignet erwiesen.

Für den Fall, dass die Zusammensetzung als Bestandteil eines Kit, wie hierin geschrieben, vorgesehen ist, kann die Konzentration der Wirksubstanz so eingestellt werden (z.B. auf 1-60 Gew.-%),dass nach Vermischen mit weiteren Kitbestandteilen die gewünschte Anwendungskonzentration erhalten wird.

Die Zusammensetzung kann weiterhin Hilfs- oder/und Zusatzstoffe umfassen und insbesondere solche Stoffe, welche in der Kosmetik oder Pharmazie üblich sind. Beispiele für derartige Substanzen sind etwa Puffer, Stabilisatoren, zusätzliche Emulagtoren, Verdickungsmittel, etc.

Ein weiterer Gegenstand der vorliegenden Erfindung ist eine erfindungsgemäße Zusammensetzung in Form eines pharmazeutischen Präparats. In diesem Fall ist die Zusammensetzung frei von Bestandteilen, die pharmazeutisch nicht akzeptabel sind und bevorzugt frei von Bestandteilen, welche beispielsweise Irritationen hervorrufen. Das pharmazeutische Präparat kann neben den bereits genannten Trägersubstanzen weitere Hilfs- oder/und Zusatzstoffe enthalten, die akzeptabel und bevorzugt gut verträglich sind.

Die Zusammensetzung kann als Lösung, Suspension, Emulsion, Mikroemulsion, Gel, Salbe, Spray, Schaum, Suppositorium oder Ovolum vorliegen.

Das pharmazeutische Präparat kann in einer Form vorliegen, die für eine systemische Verabreichung geeignet ist, wie beispielsweise eine injizierbare Flüssigkeit. Für dermatologische und gynäkologische Anwendungen liegt das Präparat jedoch bevorzugt in einer Form vor, die für eine topische Applikation geeignet ist. Das Präparat weist für die jeweils gewünschte Applikationsform günstige Eigenschaften, z.B. geeignete Viskosität, rheologische Eigenschaften, Benetzungs- und Penetrationsvermögen auf, um zu gewährleisten, dass nach der Applikation ein ausreichendes Eindringen in das Zielgewebe erfolgt. Diese genannten Eigenschaften können durch Zugabe von Verdickungs- und Benetzungsmitteln sowie Penetrationsförderern wie beispielsweise Polyethylenglykolstearylethern, Polyethylen-glykolstearaten, Polysacchariden wie etwa Polysaccharid B-1459, Softisan^{®} 378, Clofibrinsäure, 2-Pyrrolidon, Acetylcystein oder/und Carbocystein eingestellt werden.

Neben 5-ALA oder Derivaten davon kann die Zusammensetzung auch weitere Arzneistoffe enthalten, die beispielsweise aus Lokalanaesthetika, Antibiotitka, Protaglandienen, steroidalen und nichtsteroidalen Entzündungsehmmern, Wachstumshormonen, Cytokinen wie etwa TNF, Sexualhormonen oder Vitaminen ausgewählt sein können.

Zur Herstellung der erfindungsgemäßen Zusammensetzung bzw. des pharmazeutischen Präparats wird die Wirksubstanz in der nichtwässrigen Flüssigkeit gelöst oder dispergiert. Gegebenenfalls vorhandene Zusatzstoffe können vor, während oder/und nach dem Lösen bzw. Dispergieren zugesetzt werden.

Bevorzugt wird das Verfahren unter Luftausschluss durchgeführt, beispielsweise mittels Anlegen eines Vakuums oder/und einer Schutzgasatmosphäre. Außerdem ist es bevorzugt, unter Lichtausschluss zu arbeiten. Das Verfahren wird bei einer Temperatur durchgeführt, bei der die Ausbildung der gewünschten Zusammensetzung stattfinden kann und eine ausreichende Stabilität der Bestandteile, insbesondere der Wirksubstanz gegeben ist. Im allgemeinen hat sich ein Temperaturbereich von etwa 5 bis 45 °C als geeignet herausgestellt. Für eine pharmazeutische Anwendung wird für eine Sterilität des entstehenden Produkts gesorgt, z.B. durch Einsatz steriler Ausgangsmaterialien und Einhaltung steriler Verfahrensbedingungen oder/und durch einen Sterilisationsschritt nach der Herstellung.

Ein weiterer Gegenstand der vorliegenden Erfindung ist ein Kit, insbesondere zur therapeutischen oder diagnostischen Verwendung, welcher eine wie oben beschriebene 5-Aminolävulinsäure (5-ALA) oder/und ein Derivat davon enthaltende wasserfreie Zusammensetzung und getrennt davon eine wasser-haltige Zusammensetzung, insbesondere ein wässriges Puffersystem, eine wässrige Lösung oder ein wässriges Trägersystem umfasst.

Der erfindungsgemäße Kit enthält somit eine erste Komponente a), welche eine wasserfreie Zusammensetzung darstellt, in der 5-ALA oder Derivate davon gelöst oder dispergiert in einer nicht-wässrigen Flüssigkeit mit erhöhter Stabilität gelagert werden können. Davon getrennt umfasst die zweite Komponente b) des Kits ein wässriges System, insbesondere ein wässriges Puffersystem, eine wässrige Lösung oder ein wässriges Trägersystem. Die Komponente b) des Kits umfasst bevorzugt mindestens . 50 Ges.=% Wasser, mehr bevorzugt mindestens 80 Gew.-% Wasser und am meisten bevorzugt mindestens 90 Gew.-% Wasser. Bei der Komponente b) kann' es sich auch um reines Wasser handeln. Nach Vermischen der beiden in dem erfindungsgemäßen Kit seperat vorliegenden Flüssigkeiten wird eine Gemischzusammensetzung erhalten, die für die Applikation vorteilhafte Eigenschaften aufweist, während gleichzeitig eine hohe Langzeitstabilität der einzelnen Komponenten durch die Anordnung in einem Kit erzielt wird.

Durch die getrennte Bereitstellung einer 5-Aminolävulinsäure oder/und eines Derivates davon in nicht-wässriger Flüssigkeit und eines wässrigen Systems werden insbesondere folgende Vorteile erzielt: .

In der nicht-wässrigen Phase der Komponente a) liegt der Wirkstoff 5-ALA oder/und ein Derivat davon überwiegend in der Enolform vor und kann somit nicht in das dimere Dihydroxypyrazin-Derivat übergehen. Eine Reaktion mit sich selbst unter Bildung einer Schiff 'schen Base, wobei das Dehydrodxypyracin-Derivat entsteht, setzt eine Ketoform der 5-Aminolävulinsäure voraus. Während sich 5-ALA in einer wässrigen Lösung überwiegend in der Ketoform befindet, liegt es in der nicht-wässrigen Phase der Komponente a) des erfindungsgemäßen Kits überwiegend in der Enolform vor, so dass diese unerwünschte Reaktion nicht auftreten kann. Somit ist die Stabilität des Wirkstoffs im wasserfreien System, wie es in der Komponente a) des erfindungsgemäßen Kits vorliegt, deutlich erhöht. Die wasserfreie Zusammensetzung eignet sich somit als Lagerungsform für 5-ALA mit hoher Langzeitstabilität.

Trägt man aber eine wasserfreie Formulierung, wie sie als Komponente a) des erfindungsgemäßen Kits enthalten ist, direkt auf Gewerbe auf, kann beim Kontakt mit Gewebswasser ein sehr niedriger pH-Wert entstehen, welcher die Bioverfügbarkeit von 5-ALA erniedrigt. Die Kinetik der Protoporphyrin IX (PPIX) Synthese ist bei neutralem oder leicht basischem pH-Wert deutlich erhöht gegenüber sauren Umgebungsbedingungen. Weiterhin kann es bei direktem Auftrag aufgrund des niedrigen pH-Wertes zu einer nicht gewollten Zellschädigung in gesundem Gewebe kommen. Damit könnte ein Teil der Selektivität der photodynamischen Therapie (PDT) verloren gehen, die gerade dann eingesetzt wird, wenn selektiv nur veränderte, also nicht gesunde Zellen, geschädigt oder zerstört werden sollen.

Die Komponente b) des erfindungsgemäßen Kits dient der pH-Wert-Einstellung und erhöht somit sowohl die Bioverfügbarkeit des Wirkstoffes als auch die Selektivität der Zellschädigung bei einer photodynamischen Therapie.

Die Komponente b) enthält deshalb bevorzugt eine wässrige Lösung, die geeignet ist, in der Mischung zwischen den Komponenten a) und b) einen physiologischen pH-Wert einzustellen. Das wässrige System der Komponente b) weist bevorzugt einen pH-Wert > 7, mehr bevorzugt einen pH-Wert > 8 und am meisten bevorzugt einen pH-Wert > 9 auf. Bei der wasser-enthaltenden Zusammensetzung der Komponente b) handelt es sich bevorzugt um eine wässrige Lösung, insbesondere eine alkalische Lösung, um ein wässriges Puffersystem oder/und um ein wässriges Trägersystem, welches neben Wasser Hilfsstoffe, beispielsweise Applikationshilfsstoffe umfasst. Bevorzugt handelt es sich bei der wasser-enthaltenden Zusammensetzung um eine NaOH- oder KOH-Lösung oder um einen Phosphat- oder Carbonat-Pufter, bevorzugt mit geeigneter Kapazität, um den pH-Wert nach Vereinigung mit der Komponente a) auf einen pH-Wert im neutralen oder leicht alkalischen Bereich einzustellen. Bevorzugt wird ein pH-Wert von ≥ 5, mehr bevorzugt von ≥ 6, noch mehr bevorzugt von ≥ 7 und am meisten bevorzugt von ≥ 8 eingestellt.

Besonders bevorzugt ist der erfindungsgemäße Kit für eine topische Applikation vorgesehen. Die Kombination der beiden Kit-Komponenten erfolgt bevorzugt kurz vor der Applikation, beispielsweise 10 sec bis 1 h vor der Applikation, kann aber auch bei der Applikation erfolgen.

Durch den erfindungsgemäßen Kit können sowohl die Lagereigenschaften als auch die Applikationseigenschaften einer 5-ALA als Wirkstoff enthaltenden Zusammensetzung optimiert werden.

Der erfindungsgemäße Kit liegt bevorzugt in Form eines Doppelkammersystems vor, wobei die Komponente a) in einer ersten Kammer des Systems und die Komponente b) in einer zweiten Kammer des . Systems angeordnet ist. Kurz vor der Applikation oder bei der Applikation werden die beiden Kammerinhalte vermischt, um einen pH-Wert im neutralen oder leicht alkalischen Bereich einzustellen.

Die Erfindung betrifft somit insbesondere ein 2-Kammersystem, welches Zusammensetzugen der 5-Aminolävulinsäure und/oder Derivate enthält, in welchen die Wirkstoffe in einer nicht-wässrigen Phase gelagert werden und welche nach Mischung mit einer wässrigen Phase zur Anwendung gelangen.

Ein wesentliches Verwendungsgebiet für die erfindungsgemäßen Zusammensetzungen und Kits liegt auf dem Gebiet der photodynamischen Therapie, wobei die Zusammensetzung besonders bevorzugt topisch oder systemisch appliziert wird. Der Einsatz der erfindungsgemäßen Zusammensetzung ist weiterhin möglich bei sämtlichen Erkrankungen, deren Bekämpfung eine Proliferationshemmung oder eine Abtötung von Zellen oder Gewebe mittels Photoaktivierung eines aus 5-ALA gebildeten Sensibilisators umfasst. Hierzu zählen insbesondere Erkrankungen, die mit einer gesteigerten Zellproliferation assoziiert sind, da in diesem Fall eine besonders hohe Anreicherung des Photosensibilisators durch den gesteigerten Zellmetabilismus in erkrankten Zellen stattfindet.

Die erfindungsgemäßen Zusammensetzungen und Kits sind demnach geeignet zur Behandlung von Tumorerkrankungen, wie beispielsweise Basalzellkarzinome, Plattenepithelkarzinome, Morbus Bowen, aktinische Keratose, Condylomata acuminata (CIN), epitheliale Neoplasie der Vulva (VIN), knotenartige und subkutane Krebserkrankungen. Ein Beispiel für eine nichttumorartige Erkrankung ist etwa Psoriasis oder Akne.

Die Behandlung erfolgt z.B. durch topische Applikation einer die Wirksubstanz, z.B. 5-ALA enthaltenden Zusammensetzung und anschließende Inkubation, um das Eindringen einer ausreichenden Menge der 5-ALA in das zu behandelnde Gewebe zu gestatten. Während der Inkubation wird eine Lichteinstrahlung auf die behandelte Stelle bevorzugterweise z.B. durch Abdecken vermieden, um eine unerwünschte vorzeitige Aktivierung zu verhindern. Nach Ablauf des Inkubationszeitraumes, der im Allgemeinen etwa 1 bis 8 h, und üblicherweise etwa 4 h beträgt, wird das Gewebe mit einer Lichtquelle in einer ausreichenden Strahlungsdosis bestrahlt. Geeignete Lichtquellen umfassen Lampen, welche weißes Licht abgeben, sowie monochromatische Lichtquellen, wie etwa einen Laser, insbesondere Argon-Farbstofflaser mit einer Emission bei etwa 630 nm. Die Strahlungsdosen liegen üblicherweise in einem Bereich von etwa 20 J/cm² bis mehrere 100 J/cm² pro Anwendung.

Ein weiteres Verwendungsgebiet für die erfindungsgemäßen Zusammensetzungen betrifft den Nachweis des Vorhandenseins von proliferierenden Zellen in einer Probe, z.B. einer Gewebeprobe. Der Nachweis beruht auf einer selektiven Anreicherung eines durch Metabolisierung der Wirksubstanz erzeugten Photosensibilisators in den proliferierenden Zellen, verglichen mit normalen Zellen. Vorzugsweise ist die Wirksubstanz 5-ALA und der Photosensibilisator Protoporphyrin IX. Die Anreicherung des Photosensibilisators kann durch photodiagnostische Verfahren bestimmt werden, z.B. durch Bestrahlen mit Licht mit 405 nm Wellenlänge und Messen der durch den Photosensiblisator erzeugten Fluoreszenzstrahlung. Die erfindungsgemäßen Zusammensetzungen sind insbesondere zur Verwendung in der Tumordiagnostik geeignet.

Ein weiterer Gegenstand der Erfindung ist die Verwendung der erfindungsgemäßen Zusammensetzung oder/und des erfindungsgemäßen Kits zur Herstellung eines Medikaments für die photodynamische Therapie.

Schließlich betrifft die Erfindung einen weiteren Kit, welcher eine erfindungsgemäße, zur topischen Applikation oder zur Applikation in Körperhöhlen geeignete Zusammensetzung sowie eines oder mehrere Hilfsmittel enthält. Solche Hilfsmittel sind beispielsweise ein Abdeckmaterial, wie etwa eine Plastikfolie; das z.B. bei topischer Applikation, nicht jedoch bei Applikation in Kärperhöhlen, nach dem Aufbringen der Zusammensetzung auf die zu behandelnde Stelle aufgebracht wird, um eine vorzeitige Aktivierung durch Licht zu verhindern. Mittel zur Befestigung des Abdeckmaterials oder auch Mittel zum Auftragen der Zusammensetzung auf die zu behandelnde Stelle.

Ferner betrifft die Erfindung die Verwendung einer nicht-wässrigen Flüssigkeit mit einer Dielektrizitätskonstante von weniger als 80 bei 25 °C zur Stabilisierung von 5-Aminolävulinsäure (5-ALA) oder/und einem Derivat davon, wobei die 5-Aminolävulinsäure oder/und das Derivat davon in der nicht-wässrigen Flüssigkeit gelöst oder dispergiert wird.

Die nachfolgenden Abbildungen und Beispiele sollen die Erfindung weiterhin erläutern.

### Es zeigen:

Abbildung 1:Eine schematische Darstellung von Zersetzungsreaktionen, denen 5-Aminolävulinsäure (5-ALA) unterliegt.

Abbildung 2:Die zeitabhängige Veränderung des UV-VIS-Spektrums einer 10%igen 5-ALA Lösung in wasserfreiem Glycerin (unverdünnt und nach Verdünnen mit Wasser im Verhältnis 1:1 in Intervallen von 0,2 min aufgenommen).

### Beispiele

### 1.Herstellung nichtwässriger 5-ALA-Zusammensetzungen

Es wurden 10%ige Lösungen (Gew.-/Vol) von 5-ALA in 1,2-Propylenglykol und Glycerin hergestellt. Nach vollständiger Lösung wurde eine

Gelbfärbung gefunden, die jedoch nicht auf eine Zersetzung von 5-ALA zu einem der in Abb. 1 aufgeführten Abbauprodukte zurückzuführen ist. So konnten bei Kapillarelektrophorese weder DHPY, PY noch Porphobilinogen nachgewiesen werden.

Die Verfärbung der Lösung war demzufolge auf die Ausbildung der Enolform von 5-ALA zurückzuführen. Dies wurde durch UV-VIS-Messungen bestätigt. Sowohl in Glycerin als auch in 1,2 Propylenglykol wurde eine Absorptionsbande bei 447 nm gefunden, welche die Ursache der optisch erkennbaren Gelbfärbung war. Diese spektrale Verschiebung ist durch Enolisierung von 5-ALA bedingt, die bereits in wässrigen alkalischen Lösungen beobachtet wurde (Monteiro et al., Arch. Biochem. Biophys. 271 (1989), 206-217).

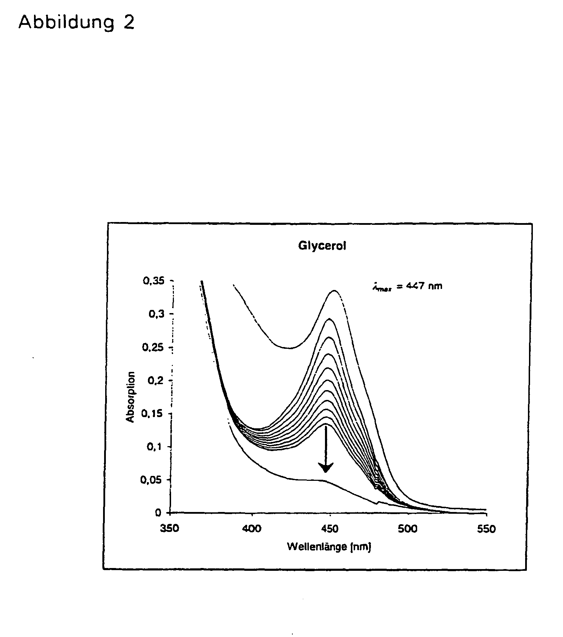

Wird der 10%igen wasserfreien 5-ALA-Lösung Wasser im Verhältnis 1:1 zugesetzt, beobachtete man ein Verschwinden der Gelbfärbung der Lösung innerhalb weniger Minuten. Dies ließ sich durch Messung der Abnahme der Extinktion bei 447 nm erfassen (Abb. 2). In einer anschließend 1:100 mit Wasser verdünnten Lösung wurde ein UV-Spektrum von 5-ALA ohne Vorhandensein von Nebenprodukten beobachtet.

## Patentansprüche

1. Wasserfreie Zusammensetzung
**dadurch gekennzeichnet,**
**dass** sie eine Wirksubstanz ausgewählt aus 5-Aminolävulinsäure (5-ALA) oder/und einem Derivat davon gelöst oder dispergiert in einer nicht-wässrigen Flüssigkeit enthält, die eine Dielektrizitätskonstante ε von weniger als 80 bei 25°C aufweist, welche eine Stabilisierung der 5-Aminolävulinsäure (5-ALA) oder/und dem Derivat davon bewirkt.

2. Zusammensetzung nach Anspruch 1,
**dadurch gekennzeichnet,**
**dass** die Derivate aus Salzen und Estern von 5-ALA ausgewählt sind.

3. Zusammensetzung nach Anspruch 1 bis 2,
**dadurch gekennzeichnet,**
**dass** die Wirksubstanz überwiegend in der Enolform vorliegt.

4. Zusammensetzung nach einem der vorhergehenden Ansprüche,
**dadurch gekennzeichnet,**
**dass** die nicht-wässrige Flüssigkeit ausgewählt ist aus Alkoholen, Ethern, Estern, Poly(alkylenglykolen), Phospholipiden, DMSO, N-Vinylpyrrolidon, N-N-Dimethylacetamid und Gemischen davon.

5. Zusammensetzung nach einem der vorhergehenden Ansprüche,
**dadurch gekennzeichnet,**
**dass** die nicht-wässrige Flüssigkeit zumindest teilweise mit Wasser mischbar ist.

6. Zusammensetzung nach einem der vorhergehenden Ansprüche,
**dadurch gekennzeichnet,**
**dass** die Wirksubstanz in einem Anteil von 1 bis 25 Gew.-%, insbesondere von 1 bis 15 Gew.-% bezogen auf das Gesamtgewicht
der Zusammensetzung vorhanden ist.

7. Zusammensetzung nach einem der vorhergehenden Ansprüche,
**dadurch gekennzeichnet,**
**dass** sie weiterhin Hilfs- oder/und Zusatzstoffe umfasst, die in der Kosmetik oder Pharmazie üblich sind.

8. Zusammensetzung nach einem der vorhergehenden Ansprüche,
**dadurch gekennzeichnet,**
**dass** sie als Lösung Suspension, Emulsion, Mikroemulsion, Gel, Salbe, Spray, Schaum, Suppositorium oder Ovolum vorliegt.

9. Zusammensetzung nach einem der vorhergehenden Ansprüche,
**dadurch gekennzeichnet,**
**dass** sie in Form eines pharmazeutischen Präparats ist.

10. Zusammensetzung nach Anspruch 9,
**dadurch gekennzeichnet,**
**dass** sie topisch applizierbar ist..

11. Zusammensetzung nach einem der vorhergehenden Ansprüche,
**dadurch gekennzeichnet,**
**dass** sie einen weiteren Arzneistoff enthält.

12. Kit, insbesondere zur therapeutischen oder diagnostischen Verwendung, umfassend
a) eine 5-Aminolävulinsäure (5-ALA) oder/und ein Derivat davon enthaltende wasserfreie Zusammensetzung nach einem der Ansprüche 1 bis 11 und
b) eine Wasser enthaltende Zusammensetzung.

13. Kit nach Anspruch 12,
**dadurch gekennzeichnet,**
**dass** die Komponente b) eine wässrige Lösung enthält, die geeignet ist in der Mischung zwischen den Komponenten a) und b) einen physiologischen pH-Wert einzustellen.

14. Kit nach einem der Ansprüche 12 bis 13,
**dadurch gekennzeichnet,**
**dass** die Komponente b) eine NaOH- oder KOH-Lösung oder/und einen Phosphat- oder/und Carbonat-Puffer enthält.

15. Kit nach einem der Ansprüche 12 bis 14,
**dadurch gekennzeichnet,**
**dass** er in Form eines Doppelkammersystems vorliegt, wobei die Komponente a) in einer ersten Kammer des Systems und die Komponente b) in einer zweiten Kammer des Systems angeordnet ist.

16. Verwendung einer eine Wirksubstanz ausgewählt aus 5-ALA, oder
einem Derivat davon enthaltenden Zusammensetzung nach einem der Ansprüche 1 bis 11 oder eines Kits nach einem der Ansprüche 12 bis 15 zur Herstellung eines Arzneimittels zur Therapie oder Diagnostik von mit Zellproliferation assoziierten Erkrankungen.

17. Verwendung nach Anspruch 16,
**dadurch gekennzeichnet,**
**dass** die Zusammensetzung topisch oder systemisch appliziert wird.

18. Verwendung nach Anspruch 16 oder 17 bei der Therapie oder Diagnostik von Tumorerkrankungen.

19. Verwendung nach einem der Ansprüche 16 bis 18 bei einer photodynamischen Therapie.

20. Verwendung nach Anspruch 18 oder 19,
**dadurch gekennzeichnet,**
**dass** die Erkrankung ein Basalzellkarzinom, ein Plattenepithelkarzinom, Morbus bowen, aktinische Keratose, Condyomata acuminata (CIN), intraepitheliale Neoplasie der Vulva (VIN) oder eine knotenartige oder subkutane Krebserkrankung ist.

21. Verwendung nach Anspruch 20,
**dadurch gekennzeichnet,**
**dass** die Erkrankung Psoriasis oder Akne ist.

22. Verwendung einer Zusammensetzung nach einem der Ansprüche 1 bis 11 oder eines Kits nach einem der Ansprüche 12 bis 15 zur Herstellung eines Diagnostikums oder Medikaments für die photodynamische Therapie.

23. Kit, umfassend eine topisch applizierbare Zusammensetzung nach Anspruch 10 und mindestens eine Komponente, ausgewählt aus
(a) einem im wesentlichen lichtundurchlässigen blattartigen Material,
(b) Mittel zur Befestigung des blattartigen Materials auf einem Applikationsort, und
(c) Mittel zum Auftragen der Zusammensetzung auf einem Applikationsort.

24. Kit nach Anspruch 23,
weiterhin umfassend
(d) ein wässriges Puffersystem.

25. Verwendung einer nicht-wässrigen Flüssigkeit mit einer Dielektrizitätskonstante von weniger als 80 bei 25 °C zur Stabilisierung von 5-Aminolävulinsäure (5-ALA) oder/und einem Derivat davon, wobei die 5-Aminolävulinsäure oder/und das Derivat davon in der nicht-wässrigen Flüssigkeit gelöst oder dispergiert wird.

## Claims

1. Anhydrous composition,
**characterized in that**
it contains an active substance selected from 5-aminolevulinic acid (5-ALA) or/and a derivative thereof dissolved or dispersed in a non-aqueous liquid which has a dielectric constant ε of less than 80 at 25°C which stabilizes the 5-aminolevulinic acid (5-ALA) or/and the derivative thereof.

2. Composition according to claim 1,
**characterized in that**
the derivatives are selected from salts and esters of 5-ALA.

3. Composition according to claim 1 or 2,
**characterized in that**
the active substance is mainly present in the enol form.

4. Composition according to one of the previous claims,
**characterized in that**
the non-aqueous liquid is selected from alcohols, ethers, esters, poly(alkylene glycols), phospholipids, DMSO, N-vinylpyrrolidone, N-N-dimethyl acetamide and mixtures thereof.

5. Composition according to one of the previous claims,
**characterized in that**
the non-aqueous liquid is at least partially miscible with water.

6. Composition according to one of the previous claims,
**characterized in that**
the active substance is present in an amount of 1 to 25 % by weight, in particular of 1 to 15 % by weight relative to the total weight of the composition.

7. Composition according to one of the previous claims,
**characterized in that**
it additionally contains auxiliary substances or/and additives that are commonly used in cosmetics or pharmaceuticals.

8. Composition according to one of the previous claims,
**characterized in that**
it is present as a solution, suspension, emulsion, microemulsion, gel, ointment, spray, foam, suppository or ovulum.

9. Composition according to one of the previous claims,
**characterized in that**
it is in the form of a pharmaceutical preparation.

10. Composition according to claim 9,
**characterized in that**
it can be applied topically.

11. Composition according to one of the previous claims,
**characterized in that**
it contains an additional medicinal substance.

12. Kit in particular for therapeutic or diagnostic use, comprising
a) an anhydrous composition containing 5-aminolevulinic acid (5-ALA) or/ and a derivative thereof according to one of the claims 1 to 11 and
b) a composition containing water.

13. Kit according to claim 12,
**characterized in that**
component b) contains an aqueous solution which is suitable for adjusting a physiological pH in the mixture of components a) and b).

14. Kit according to one of the claims 12 to 13,
**characterized in that**
component b) contains an NaOH or KOH solution or/and a phosphate or/and carbonate buffer.

15. Kit according to one of the claims 12 to 14,
**characterized in that**
it is present in the form of a double-chamber system in which component a) is located in a first chamber of the system and component b) is located in a second chamber of the system.

16. Use of a composition containing an active substance selected from 5-ALA or a derivative thereof according to one of the claims 1 to 11 or of a kit according to one of the claims 12 to 15 to produce a pharmaceutical preparation for the treatment or diagnosis of diseases associated with cell proliferation.

17. Use according to claim 16,
**characterized in that**
the composition is applied topically or systemically.

18. Use according to claim 16 or 17 for the treatment or diagnosis of tumour diseases.

19. Use according to one of the claims 16 to 18 for a photodynamic therapy.

20. Use according to claim 18 or 19,
**characterized in that**
the disease is a basal cell carcinoma, squamous-cell carcinoma, morbus Bowen, actinic keratosis, condylomata acuminata (CIN), intraepithelial neoplasia of the vulva (VIN) or a nodular or subcutaneous cancer disease.

21. Use according to claim 20,
**characterized in that**
the disease is psoriasis or acne.

22. Use of a composition according to one of the claims 1 to 11 or of a kit according to one of the claims 12 to 15 for producing a diagnostic agent or medicament for photodynamic therapy.

23. Kit comprising a composition that can be applied topically according to claim 10 and at least one component selected from
(a) an essentially light-impermeable sheet-like material,
(b) means for attaching the sheet-like material to a site of application and
(c) means for applying the composition to a site of application.

24. Kit according to claim 23,
additionally comprising
(d) an aqueous buffer system.

25. Use of a non-aqueous liquid having a dielectric constant of less than 80 at 25°C for stabilizing 5-aminolevulinic acid (5-ALA) or/and a derivative thereof wherein the 5-aminolevulinic acid or/and the derivative thereof is dissolved or dispersed in the non-aqueous liquid.

## Revendications

1. Composition anhydre **caractérisée en ce qu'**elle contient une substance active choisie parmi l'acide 5-aminolévulinique (5-ALA) et/ou un dérivé de celui-ci dissoute ou dispersée dans un liquide non aqueux qui présente une constante diélectrique ε inférieure à 80 à 25°C qui entraîne une stabilisation de l'acide 5-aminolévulinique (5-ALA) et/ou de son dérivé.

2. Composition selon la revendication 1 **caractérisée en ce que** les dérivés sont choisis parmi les sels et esters de 5-ALA.

3. Composition selon les revendications 1 à 2 **caractérisée en ce que** la substance active est principalement sous la forme énol.

4. Composition selon l'une des revendications précédentes **caractérisée en ce que** le liquide non aqueux est choisi parmi les alcools, les éthers, les esters, les poly(alkylèneglycols), les phospholipides, le DMSO, la N-vinylpyrrolidone, le N,N-diméthylacétamide et leurs mélanges.

5. Composition selon l'une des revendications précédentes **caractérisée en ce que** le liquide non aqueux est au moins en partie miscible à l'eau.

6. Composition selon l'une des revendications précédentes **caractérisée en ce que** la substance active est présente en une proportion de 1 à 25 % en poids, en particulier de 1 à 15 % en poids par rapport au poids total de la composition.

7. Composition selon l'une des revendications précédentes **caractérisée en ce qu'**elle comprend en outre des adjuvants et/ou additifs qui sont courants en cosmétique ou en pharmacie.

8. Composition selon l'une des revendications précédentes **caractérisée en ce qu'**elle est sous forme de solution, de suspension, d'émulsion, de microémulsion, de gel, de pommade, de spray, de mousse, de suppositoire ou d'ovule.

9. Composition selon l'une des revendications précédentes **caractérisée en ce qu'**elle est sous forme d'une préparation pharmaceutique.

10. Composition selon la revendication 9 **caractérisée en ce qu'**elle est applicable par voie topique.

11. Composition selon l'une des revendications précédentes **caractérisée en ce qu'**elle contient une autre substance médicamenteuse.

12. Kit, en particulier pour l'utilisation thérapeutique ou diagnostique, comprenant
a) une composition anhydre contenant de l'acide 5-aminolévulinique (5-ALA) et/ ou un dérivé de celui-ci selon l'une des revendications 1 à 11 et
b) une composition contenant de l'eau.

13. Kit selon la revendication 12 **caractérisé en ce que** le composant b) contient une solution aqueuse qui convient pour établir un pH physiologique dans le mélange entre les composants a) et b).

14. Kit selon l'une des revendications 12 à 13 **caractérisé en ce que** le composant b) contient une solution de NaOH ou KOH et/ou un tampon phosphate et/ou carbonate.

15. Kit selon l'une des revendications 12 à 14 **caractérisé en ce qu'**il est sous forme d'un système à deux chambres où le composant a) est disposé dans une première chambre du système et le composant b) est disposé dans une seconde chambre du système.

16. Utilisation d'une composition contenant une substance active choisie parmi 5-ALA et un dérivé de celui-ci selon l'une des revendications 1 à 11 ou d'un kit selon l'une des revendications 12 à 15 pour la production d'un produit médicamenteux pour la thérapie ou le diagnostic des maladies associées avec la prolifération cellulaire.

17. Utilisation selon la revendication 16 **caractérisée en ce que** la composition est appliquée par voie topique ou systémique.

18. Utilisation selon la revendication 16 ou 17 dans la thérapie ou le diagnostic des maladies tumorales.

19. Utilisation selon l'une des revendications 16 à 18 dans une thérapie photodynamique.

20. Utilisation selon la revendication 18 ou 19 **caractérisée en ce que** la maladie est un carcinome basocellulaire, un carcinome d'épithélium pavimenteux, la maladie de Bowen, la kératose actinique, le condylome acuminé (CIN), la néoplasie intraépithéliale vulvaire (VIN) ou une maladie cancéreuse nodulaire ou sous-cutanée.

21. Utilisation selon la revendication 20 **caractérisée en ce que** la maladie est le psoriasis ou l'acné.

22. Utilisation d'une composition selon l'une des revendications 1 à 11 ou d'un kit selon l'une des revendications 12 à 15 pour la production d'un agent de diagnostic ou d'un médicament pour la thérapie photodynamique.

23. Kit comprenant une composition applicable par voie topique selon la revendication 10 et au moins un composant choisi parmi
(a) un matériau de type feuille sensiblement imperméable à la lumière,
(b) des moyens pour fixer le matériau de type feuille sur un site d'application, et
(c) des moyens pour appliquer la composition sur un site d'application.

24. Kit selon la revendication 23 comprenant en outre
(d) un système tampon aqueux.

25. Utilisation d'un liquide non aqueux ayant une constante diélectrique inférieure à 80 à 25°C pour la stabilisation de l'acide 5-aminolévulinique (5-ALA) et/ou d'un dérivé de celui-ci, où l'acide 5-aminolévulinique et/ou son dérivé sont dissous ou dispersés dans le liquide non aqueux.
